# EUROPEAN PATENT APPLICATION

(11) **EP 4 835 084 A1**
(43) Date of publication of application: **04.11.2026**
(21) Application number: 26734782.1
(22) Date of filing: 25.02.2026
(51) Int. Cl.: C12N 15/77, C12N 9/24, C12P 13/08

(54) **MICROORGANISM HAVING TREHALASE INTRODUCED THEREINTO AND USES THEREOF**

(30) Priority: 26.02.2025 KR 20250025095
(71) Applicant: CJ CHEILJEDANG CORPORATION, Seoul 04560 (KR)
(72) Inventor: KIM, Hye Mi, Seoul 04560 (KR); SHIN, Kwang Soo, Seoul 04560 (KR); SON, Byung-Sam, Seoul 04560 (KR); LEE, Hyein, Seoul 04560 (KR)
(74) Representative: Meissner Bolte Partnerschaft mbB
(86) International application number: PCT/KR2026/003143
(87) International publication number: WO 2026/182536

(57) **Abstract**

The present disclosure provides a trehalase-introduced microorganism and a use thereof.

## Description

### [TECHNICAL FIELD]

The present disclosure relates to a trehalase-introduced microorganism and a use thereof.

### Cross-Reference to Related Application(s)

This application claims the benefit of priority based on Korean Patent Application No. 10-2025-0025095, filed on February 26, 2025, the entire contents of which are incorporated herein by reference.

### [BACKGROUND ART]

L-lysine is an essential amino acid and is essential for physiological functions in humans and animals. In particular, in the feed and food industry, lysine is used as an important component of protein synthesis. Lysine is mainly produced through a fermentation process using a microorganism. Since lysine productivity highly depends on the carbon metabolism capability of the microorganism, efficient use of an energy source acts as an important factor in productivity. Therefore, research for an effective method for increasing lysine production is required.

### [Prior Art Documents]

(Patent Document 1) US Patent Publication No. 2011-0008864 A1

### [DETAILED DESCRIPTION OF THE INVENTION]

### [TECHNICAL PROBLEM]

One object of the present disclosure is to provide a lysine-producing microorganism of the genus *Corynebacterium*, into which an E. coli-derived trehalase is introduced.

Another object of the present disclosure is to provide a composition for producing lysine comprising the microorganism of the genus *Corynebacterium*.

Still another object of the present disclosure is to provide a method for producing lysine, comprising culturing the microorganism of the genus *Corynebacterium* in a medium.

Still another object of the present disclosure is to provide a method for increasing lysine production, comprising culturing the microorganism of the genus *Corynebacterium* in a medium.

### [TECHNICAL SOLUTION]

This will be described in detail as follows. Meanwhile, each description and embodiment disclosed in the present disclosure may be applied to each of the other descriptions and embodiments. That is, all combinations of various elements disclosed in the present disclosure fall within the scope of the present disclosure. In addition, the scope of the present disclosure may not be considered to be limited by the specific descriptions described below. In addition, a number of papers and patent documents are referenced throughout this specification and their citations are indicated. The disclosures of the cited papers and patent documents are incorporated herein by reference in their entirety to more clearly explain the level of the technical field to which the present invention belongs and the contents of the present invention. In addition, a person skilled in the art may recognize or identify a number of equivalents to specific aspects of the present disclosure described in the present disclosure using only routine experimentation. In addition, such equivalents are intended to be included in the present disclosure.

Hereinafter, the present disclosure will be described in more detail.

One aspect provides a lysine-producing microorganism of the genus *Corynebacterium*, into which an Escherichia coli (E. coli)-derived trehalase is introduced.

In the present disclosure, trehalase may refer to an enzyme that degrades trehalose into two molecules of glucose. The trehalase may be cytoplasmic trehalase (treF) and/or periplasmic trehalase (treA).

The cytoplasmic trehalase is mainly distributed in the cytoplasm of a microorganism and may degrade intracellular trehalose into glucose to supply it as an energy source necessary for the metabolism of the microorganism.

The periplasmic trehalase is mainly distributed in the periplasm, which is a space between the cytoplasmic membrane (inner membrane) and the outer membrane of a microorganism, and may degrade extracellular trehalose to be used as an energy source for the microorganism.

The amino acid sequence of the E. coli-derived trehalase or the nucleic acid sequence encoding the same may be obtained from NCBI, a known database.

In one embodiment, the E. coli-derived cytoplasmic trehalase may refer to an E. coli-derived protein having cytoplasmic trehalase activity.

In one embodiment, the E. coli-derived cytoplasmic trehalase may comprise, have, consist of, or consist essentially of the amino acid sequence of SEQ ID NO: 1 or an amino acid sequence having at least 60%, 65%, 70%, 75%, 76%, 77%, 78%, 79%, 80%, 81%, 82%, 83%, 84%, 85%, 86%, 87%, 88%, 89%, 90%, 90.5%, 91%, 91.5%, 92%, 92.5%, 93%, 93.5%, 93.8%, 93.9%, 94%, 94.5%, 95%, 95.5%, 96%, 96.5%, 97%, 97.4%, 97.5%, 97.6%, 97.7%, 97.8%, 97.9%, 98%, 98.1%, 98.2%, 98.3%, 98.4%, 98.5%, 98.6%, 98.7%, 98.8%, 98.9%, 99%, 99.1%, 99.2%, 99.3%, 99.4%, 99.5%, 99.6%, 99.7%, 99.8%, or 99.9% sequence homology or sequence identity to the amino acid sequence of SEQ ID NO: 1, but is not limited thereto.

In one embodiment, the E. coli-derived cytoplasmic trehalase may be one encoded by a gene comprising the nucleic acid sequence of SEQ ID NO: 2.

In one embodiment, the microorganism of the genus *Corynebacterium* may be one into which an E. coli-derived periplasmic trehalase is further introduced.

In one embodiment, the E. coli-derived periplasmic trehalase may refer to an E. coli-derived protein having periplasmic trehalase activity.

In one embodiment, the E. coli-derived periplasmic trehalase may comprise, have, consist of, or consist essentially of the amino acid sequence of SEQ ID NO: 3 or an amino acid sequence that has periplasmic trehalase activity and has at least 60%, 65%, 70%, 75%, 76%, 77%, 78%, 79%, 80%, 81%, 82%, 83%, 84%, 85%, 85.5%, 86%, 86.5%, 87%, 87.5%, 88%, 88.5%, 89%, 89.5%, 90%, 90.5%, 91%, 91.5%, 92%, 92.5%, 93%, 93.5%, 94%, 94.5%, 95%, 95.5%, 96%, 96.4%, 96.5%, 97%, 97.5%, 97.6%, 97.7%, 97.8%, 97.9%, 98%, 98.1%, 98.2%, 98.3%, 98.4%, 98.5%, 98.6%, 98.7%, 98.8%, 98.9%, 99%, 99.1%, 99.2%, 99.3%, 99.4%, 99.5%, 99.6%, 99.7%, 99.8%, or 99.9% sequence homology or sequence identity to the amino acid sequence of SEQ ID NO: 3, but is not limited thereto.

In one embodiment, the E. coli-derived periplasmic trehalase may be encoded by a gene comprising the nucleic acid sequence of SEQ ID NO: 4.

In addition, the trehalase may also include variants having an amino acid sequence with a deletion, modification, substitution, conservative substitution, or addition of a portion of the sequence, provided that the variant has such homology or identity and exhibits efficacy corresponding to that of a trehalase (e.g., cytoplasmic trehalase and/or periplasmic trehalase). For instance, this includes cases involving sequence addition or deletion that does not alter trehalase activity at the N-terminus, C-terminus, and/or within the amino acid sequence, or involving a naturally occurring mutation, a silent mutation, or a conservative substitution.

The "conservative substitution" refers to substituting one amino acid with another amino acid having similar structural and/or chemical properties. Such amino acid substitutions may generally occur based on similarities in polarity, charge, solubility, hydrophobicity, hydrophilicity, and/or the amphipathic nature of residues. Typically, conservative substitutions may have little or no effect on the activity of a protein or polypeptide.

In the present disclosure, the expression that a polynucleotide or polypeptide "has, comprises, consists of, or consists essentially of a specific nucleic acid sequence (or nucleotide sequence) or amino acid sequence" may be interpreted as including a "substantially equivalent sequence" in which a mutation (deletion, substitution, modification, and/or addition) is introduced into the specific nucleic acid sequence or amino acid sequence within a range that maintains the original function and/or desired function of the polynucleotide or polypeptide (or as not excluding such mutation). In one embodiment, the expression that a polynucleotide or polypeptide "has, comprises, consists of, or consists essentially of a specific nucleic acid sequence or amino acid sequence" may mean that the polynucleotide or polypeptide (i) comprises the specific nucleic acid sequence or amino acid sequence, or (ii) consists of or essentially comprises a nucleic acid sequence or amino acid sequence having at least 60%, 65%, 70%, 75%, 76%, 77%, 78%, 79%, 80%, 81%, 82%, 83%, 84%, 85%, 86%, 87%, 88%, 89%, 90%, 90.5%, 91%, 91.5%, 92%, 92.5%, 93%, 93.5%, 94%, 94.5%, 95%, 95.5%, 96%, 96.5%, 97%, 97.5%, 97.6%, 97.7%, 97.8%, 97.9%, 98%, 98.1%, 98.2%, 98.3%, 98.4%, 98.5%, 98.6%, 98.7%, 98.8%, 98.9%, 99%, 99.1%, 99.2%, 99.3%, 99.4%, 99.5%, 99.6%, 99.7%, 99.8%, or 99.9% homology or identity to the specific nucleic acid sequence or amino acid sequence, and maintains the original function and/or desired function.

In the present disclosure, "homology" or "identity" refers to the degree of similarity between two given amino acid sequences or nucleic acid sequences and may be expressed as a percentage. The terms homology and identity may often be used interchangeably.

The sequence homology or identity of a conserved polynucleotide or polypeptide is determined by a standard alignment algorithm, and a default gap penalty established by the program used may be used therewith. Substantially, homologous or identical sequences may generally hybridize with the whole or a part of the sequence under medium or highly stringent conditions. It is obvious that hybridization also includes hybridization with a polynucleotide containing a general codon or a codon taking into account codon degeneracy in the polynucleotide.

Whether any two polynucleotide or polypeptide sequences have homology, similarity, or identity may be determined using a known computer algorithm such as the "FASTA" program using default parameters as in, for example, Pearson et al (1988) [Proc. Natl. Acad. Sci. USA 85]: 2444. Alternatively, it may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including the GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, homology, similarity, or identity may be determined using BLAST of the National Center for Biotechnology Information or ClustalW.

The homology, similarity, or identity of a polynucleotide or polypeptide may be determined by comparing sequence information using a GAP computer program, as known in, for example, Smith and Waterman, Adv. Appl. Math (1981) 2:482, such as, for example, Needleman et al. (1970), J Mol Biol. 48:443. In summary, the value calculated by the GAP program may be defined as the number of similarly arranged symbols (i.e., nucleotides or amino acids) divided by the total number of symbols in the shorter of the two sequences. Default parameters for the GAP program may include (1) a binary comparison matrix (containing a value of 1 for identity and 0 for non-identity) and a weighted comparison matrix of Gribskov et al. (1986) Nucl. Acids Res. 14: 6745 (or EDNAFULL (EMBOSS version of NCBI NUC4.4) substitution matrix), as disclosed by Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or a gap opening penalty of 10, a gap extension penalty of 0.5); and (3) no penalty for terminal gaps.

In one embodiment, the microorganism of the genus *Corynebacterium* may be one or more selected from the group consisting of *Corynebacterium glutamicum*, *Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium pollutisoli, Corynebacterium imitans, Corynebacterium testudinoris*, and *Corynebacterium flavescens*, but is not limited thereto.

The microorganism of the genus *Corynebacterium* may include both a wild-type microorganism and a microorganism in which genetic modification has occurred naturally or artificially. The microorganism of the genus *Corynebacterium* is a microorganism in which a specific mechanism is strengthened or weakened due to causes such as insertion of a foreign gene or strengthening or weakening of endogenous gene activity, and may be a recombinant microorganism including a genetic modification for production of a desired polypeptide, protein, or product (e.g., lysine).

The microorganism of the genus *Corynebacterium* may be one into which an E. coli-derived trehalase (cytoplasmic trehalase (treF)) and/or periplasmic trehalase (treA)) or a gene encoding the same is introduced.

The introduction of the gene encoding the E. coli-derived trehalase may be achieved by introducing into the host cell a vector in which the gene is operably linked and which may replicate and function independently of the host cell (microorganism of the genus *Corynebacterium*), or may be achieved by introducing one copy or two or more copies into the chromosome of the host cell. The introduction into the chromosome may be performed by introducing a vector capable of inserting the polynucleotide into the chromosome into the host cell, but is not limited thereto.

The gene encoding the E. coli-derived trehalase may be inserted into a position that does not affect the expression of other genes of the host cell, for example, within genomic safe harbors. In one embodiment, the genomic safe harbors may be the NCgl2284 gene region of *Corynebacterium glutamicum*, a region between the NCgl2195 gene and the NCgl2196 gene of *Corynebacterium glutamicum*, or a region between the NCgl0866 gene and the NCgl0867 gene of *Corynebacterium glutamicum*.

The term "vector" as used in the present disclosure refers to a DNA construct for delivering a target polynucleotide into a suitable host or host cell. The vector may be a recombinant vector or an expression vector. As an example, it may comprise a nucleotide sequence of a polynucleotide encoding a target polypeptide operably linked to a suitable expression control region (or expression control sequence) so that the target polypeptide may be expressed in a suitable host. The expression control region may include a promoter capable of initiating transcription, any operator sequence for controlling such transcription, a sequence encoding a suitable mRNA ribosome binding site, and a sequence controlling termination of transcription and translation. After the vector is transformed into a suitable host cell, it may replicate or function independently of the host genome, or may be integrated into the genome itself. For example, the target polynucleotide may be inserted into the chromosome through a chromosome insertion vector. The insertion of the polynucleotide into the chromosome may be performed by any method known in the art, for example, homologous recombination, but is not limited thereto. It may further comprise a selection marker for confirming whether the polynucleotide is inserted into the chromosome. The selection marker is for selecting cells transformed with the vector, that is, for confirming whether a target nucleic acid molecule is inserted, and markers that impart a selectable phenotype such as drug resistance, auxotrophy, resistance to cytotoxic agents, or expression of a surface polypeptide may be used. In an environment treated with a selective agent, only cells expressing the selection marker survive or show other phenotypic traits, so the transformed cells may be selected.

The vector used in the present disclosure is not particularly limited, and any vector known in the art may be used. Examples of commonly used vectors include natural or recombinant plasmids, cosmids, viruses, and bacteriophages. For example, pWE15, M13, MBL3, MBL4, IXII, ASHII, APII, t10, t11, Charon4A, and Charon21A may be used as a phage vector or cosmid vector, and pDZ-based, pDC-based, pBR-based, pUC-based, pBluescriptII-based, pGEM-based, pTZ-based, pCL-based, and pET-based vectors may be used as a plasmid vector. As an example, pDZ, pDC, pACYC177, pACYC184, pCL, pECCG117, pUC19, pBR322, pMW118, and pCC1BAC vectors may be used.

Introduction of the vector into a microorganism may be performed by a person skilled in the art appropriately selecting a known transformation method. The transformation method may be performed by any method of introducing a nucleic acid into a host cell (microorganism), and may be performed by appropriately selecting a transformation technique known in the art according to the host cell. Examples of the known transformation methods include, but are not limited to, electroporation, calcium phosphate (CaPO4) precipitation, calcium chloride (CaCl2) precipitation, microinjection, polyethylene glycol-mediated uptake, the DEAE-dextran method, the cationic liposome method, lipofection, the lithium acetate-DMSO method, and genome editing using an engineered nuclease (e.g., CRISPR-Cas9).

The microorganism of the genus *Corynebacterium* into which the E. coli-derived trehalase is introduced may be one in which the activity of trehalase is enhanced by operably linking a strong promoter to a gene encoding the trehalase. That a strong promoter is operably linked to a gene encoding trehalase as described above may mean replacing (substituting) the original promoter of the trehalase with a strong promoter, or positioning the strong promoter in front of the gene encoding trehalase so that the expression of the gene encoding trehalase may be controlled by the strong promoter. The original promoter of the trehalase may refer to a promoter operably linked to the trehalase in the chromosome of a wild-type microorganism.

In the present disclosure, the term "operably (operatively) linked" means that the promoter of the present disclosure is functionally connected to the target gene sequence (e.g., a gene encoding trehalase) so as to initiate and mediate transcription of the gene. Operable linkage may be prepared using gene recombination techniques known in the technical field of the present disclosure, and site-specific DNA cleavage and ligation may be performed using cleavage and ligation enzymes, etc., known in the technical field of the present disclosure.

The strong promoter may be a promoter of an endogenous gene or a foreign gene of a microorganism. In one embodiment, the strong promoter may be derived from a microorganism of the genus *Corynebacterium*. In one specific embodiment, the strong promoter may be derived from *Corynebacterium glutamicum*.

In one embodiment, the strong promoter may be CJ1 to CJ7 promoters (US Patent No. 7662943 B2), lac promoter, trp promoter, trc promoter, tac promoter, lambda phage PR promoter, PL promoter, tet promoter, gapA promoter, SPL7 promoter, SPL13 (sm3) promoter (US Patent No. 10584338 B2), O2 promoter (US Patent No. 10273491 B2), tkt promoter, yccA promoter, etc., but is not limited thereto.

The fact that the activity of the trehalase is enhanced means that the activity of the trehalase is increased compared to the endogenous activity. The term 'enhancement' may be used interchangeably with terms such as 'activation', 'up-regulation', 'overexpression', and 'increase'. Herein, 'activation', 'enhancement', 'up-regulation', 'overexpression', and 'increase' may all include exhibiting an activity that was not originally possessed, or exhibiting an activity improved compared to an endogenous activity or an activity before modification. The term 'endogenous activity' refers to the activity of a specific polypeptide originally possessed by a parent strain or an unmodified microorganism before a trait is changed, when the trait changes due to genetic mutation by natural or artificial factors. This may be used interchangeably with 'activity before modification'. The fact that the activity of a polypeptide is 'enhanced', 'up-regulated', 'overexpressed', or 'increased' compared to the endogenous activity means that it is improved compared to the activity and/or concentration (expression level) of a specific polypeptide originally possessed by the parent strain or unmodified microorganism before the trait change.

The enhancement may be achieved by introducing a foreign polypeptide or through enhancement of activity and/or increase in concentration (expression level) of an endogenous polypeptide. Whether the activity of the polypeptide is enhanced may be confirmed from an increase in the activity level, the expression level of the polypeptide, or the amount of a product produced by the activity of the polypeptide.

The microorganism of the genus *Corynebacterium* into which the E. coli-derived trehalase is introduced may be a microorganism having lysine production ability or having increased lysine production ability (or production amount). The microorganism may be a microorganism that naturally lacks lysine production ability, or a microorganism in which lysine production ability is imparted or enhanced by introducing or strengthening trehalase activity in a microorganism having lysine production ability, but is not limited thereto.

That the microorganism has improved lysine production ability or has lysine production ability may mean that the microorganism has improved lysine production ability compared to a parent strain into which the E. coli-derived trehalase is not introduced, an unmodified microorganism, a cell before recombination, and/or a wild-type strain, or that lysine production ability is imparted unlike a parent strain into which the E. coli-derived trehalase is not introduced, an unmodified microorganism without lysine production ability, a cell before recombination, and/or a wild-type strain.

The microorganism may be one having improved (increased) lysine production ability compared to a microorganism into which an E. coli-derived trehalase has not been introduced, that is, an unmodified microorganism of the same species. In the present disclosure, an "unmodified microorganism" does not exclude a strain including a mutation that may naturally occur in a microorganism, and may mean a wild-type strain or a natural-type strain itself, or a strain before a trait is changed by genetic mutation due to natural or artificial factors. For example, the unmodified microorganism may mean a strain before a trehalase is introduced according to an embodiment. The "unmodified microorganism" may be used interchangeably with "strain before modification", "microorganism before modification", "non-mutant strain", "unmodified strain", "non-mutant microorganism", or "reference microorganism". In one embodiment, the unmodified microorganism, which is a target strain for comparing whether the lysine production ability is increased, may be a wild-type *Corynebacterium glutamicum* ATCC13032 strain, a strain into which one or more amino acid substitution mutations selected from the group consisting of pyc(P458S), hom(V59A), and lysC(T311I) have been introduced into the wild-type *Corynebacterium glutamicum* ATCC13032 strain, and/or a *Corynebacterium glutamicum* CJ3P strain (US 9556463 B2).

The microorganism may additionally comprise a mutation that increases lysine production, and the position of the mutation and/or the type of gene and/or protein targeted for mutation may be included without limitation as long as it increases lysine production. The recombinant cell may be used without limitation as long as it is a cell capable of transformation.

In one embodiment, the microorganism may have lysine production ability increased by about 5% or more, about 10% or more, about 11% or more, about 12% or more, about 12.5% or more, about 13% or more, about 13.5% or more, about 14% or more, about 15% or more, about 16% or more, about 17% or more, about 18% or more, about 19% or more, about 20% or more, about 25% or more, about 30% or more, about 35% or more, about 40% or more, about 50% or more, about 100% or more, about 200% or more, about 300% or more, about 400% or more, about 500% or more, about 600% or more, about 700% or more, about 800% or more, about 900% or more, or about 1,000% or more compared to the parent strain before mutation or unmodified microorganism, but is not limited thereto (the upper limit is not particularly limited but may be about 1000%).

In another embodiment, the microorganism may have a lysine production ability of about 1.1 times or more, about 1.11 times or more, about 1.12 times or more, about 1.125 times or more, about 1.13 times or more, about 1.135 times or more, about 1.14 times or more, about 1.145 times or more, about 1.15 times or more, about 1.2 times or more, about 1.25 times or more, about 1.3 times or more, about 1.35 times or more, about 1.4 times or more, about 1.45 times or more, about 1.5 times or more, about 2 times or more, about 3 times or more, about 4 times or more, about 5 times or more, about 6 times or more, about 7 times or more, about 8 times or more, about 9 times or more, or about 10 times or more (the upper limit value is not particularly limited and may be, for example, about 10 times) compared to the parent strain before mutation or the unmodified microorganism, but is not limited thereto.

In another embodiment, compared to the parent strain before mutation or the unmodified microorganism, the microorganism may have a lysine production ability increased by about 0.5 g/L or more, about 0.6 g/L or more, about 0.7 g/L or more, about 0.75 g/L or more, about 0.8 g/L or more, about 0.85 g/L or more, about 0.9 g/L or more, about 1.0 g/L or more, about 1.11 g/L or more, about 1.12 g/L or more, about 1.13 g/L or more, about 1.14 g/L or more, about 1.15 g/L or more, about 1.2 g/L or more, about 1.5 g/L or more, about 2.0 g/L or more, about 2.5 g/L or more, about 3 g/L or more, about 3.5 g/L or more, about 4 g/L or more, about 4.5 g/L or more, about 5 g/L or more, about 5.5 g/L or more, about 6 g/L or more, about 7 g/L or more, about 8 g/L or more, about 9 g/L or more, or about 10 g/L or more (the upper limit is not particularly limited and may be, for example, about 10 g/L), but is not limited thereto.

The microorganism may have increased trehalose degradation ability compared to the parent strain before mutation and/or the unmodified microorganism. The microorganism may improve lysine production ability because it may use more glucose by degrading trehalose with high efficiency.

In one embodiment, the microorganism may have an increased trehalose degradation ability compared to the parent strain before mutation or an unmodified microorganism, wherein the ability is increased by 40% or more, 45% or more, 50% or more, 55% or more, 60% or more, 65% or more, 70% or more, 75% or more, 80% or more, 85% or more, 90% or more, 95% or more, or in a range of 30 to 99%, 30 to 95%, 30 to 90%, 35 to 99%, 35 to 95%, 35 to 90%, 40 to 99%, 40 to 95%, 40 to 90%, 45 to 99%, 45 to 95%, 45 to 90%, 50 to 99%, 50 to 95%, 50 to 90%, 52.5 to 99%, 52.5 to 95%, 52.5 to 90%, 55 to 99%, 55 to 95%, or 55 to 90%, but is not limited thereto.

In another embodiment, the microorganism may have trehalose degradation ability as measured over the same period of time increased by 50 mg/L or more, 60 mg/L or more, 70 mg/L or more, 80 mg/L or more, 90 mg/L or more, 100 mg/L or more, 110 mg/L or more, 120 mg/L or more, 130 mg/L or more, 60 to 200 mg/L, 60 to 190 mg/L, 60 to 180 mg/L, 60 to 170 mg/L, 60 to 160 mg/L, 60 to 150 mg/L, 60 to 140 mg/L, 65 to 200 mg/L, 65 to 190 mg/L, 65 to 180 mg/L, 65 to 170 mg/L, 65 to 160 mg/L, 65 to 150 mg/L, 65 to 140 mg/L, 70 to 200 mg/L, 70 to 190 mg/L, 70 to 180 mg/L, 70 to 170 mg/L, 70 to 160 mg/L, 70 to 150 mg/L, 70 to 140 mg/L, 75 to 200 mg/L, 75 to 190 mg/L, 75 to 180 mg/L, 75 to 170 mg/L, 75 to 160 mg/L, 75 to 150 mg/L, or 75 to 140 mg/L compared to the parent strain before mutation or unmodified microorganism, but is not limited thereto.

The term "about" encompasses a range including all of ±0.5, ±0.4, ±0.3, ±0.2, ±0.1, etc., and includes all numerical values in a range equivalent or similar to the numerical value following the term "about," but is not limited thereto.

Another aspect provides a method for producing lysine or a method for increasing lysine production, comprising culturing the microorganism of the genus *Corynebacterium* in a medium.

The method for producing lysine or the method for increasing lysine production of the present disclosure may further include, for example, preparing the microorganism, preparing a medium for culturing the microorganism, or a combination thereof (in any order), before the culturing step.

The method for producing lysine or the method for increasing lysine production may include culturing the microorganism in a medium.

In the present disclosure, "culturing" may mean growing the microorganism under appropriately controlled environmental conditions. The culturing process of the present disclosure may be performed according to a suitable medium and culture conditions known in the art. Such a culturing process may be easily adjusted and used by a person skilled in the art according to the selected strain. Specifically, the culturing may be batch, continuous, and/or fed-batch, but is not limited thereto.

In the present disclosure, the term "medium" refers to a substance in which nutrients required for culturing the microorganism are mixed as main components, and may supply nutrients and growth factors, including water essential for survival and growth. Specifically, any medium and other culture conditions used for culturing the microorganism of the present disclosure may be used without particular limitation as long as it is a medium used for culturing common microorganisms, but the microorganism of the present disclosure may be cultured under aerobic conditions while controlling temperature, pH, etc. in a common medium containing a suitable carbon source, nitrogen source, phosphorus source, inorganic compound, amino acids and/or vitamins, etc. In one embodiment, a culture medium for a strain of the genus *Corynebacterium* may be found in the literature ["Manual of Methods for General Bacteriology" by the American Society for Bacteriology (Washington D.C., USA, 1981)].

The carbon source may include carbohydrates such as glucose, saccharose, lactose, fructose, sucrose, maltose, and the like; sugar alcohols such as mannitol, sorbitol, and the like; organic acids such as pyruvic acid, lactic acid, citric acid, and the like; and amino acids such as glutamic acid, methionine, lysine, and the like. In addition, natural organic nutrients such as starch hydrolysate, molasses, blackstrap molasses, rice bran, cassava, sugar cane residue, and corn steep liquor may be used; specifically, carbohydrates such as glucose and sterilized pretreated molasses (i.e., molasses converted to reducing sugar) may be used; and other appropriate amounts of carbon sources may be used in various ways without limitation. These carbon sources may be used alone or in combination of two or more, but are not limited thereto.

The nitrogen source may be selected from the group consisting of: inorganic nitrogen sources such as ammonia, ammonium sulfate, ammonium chloride, ammonium acetate, ammonium phosphate, ammonium carbonate, and ammonium nitrate; and organic nitrogen sources such as amino acids including glutamic acid, methionine, and glutamine, peptone, NZ-amine, meat extract, yeast extract, malt extract, corn steep liquor, casein hydrolysate, fish or degradation products thereof, and defatted soybean cake or degradation products thereof. These nitrogen sources may be used alone or in combination of two or more, but are not limited thereto.

The phosphorus source may include potassium phosphate monobasic, potassium phosphate dibasic, or a corresponding sodium-containing salt. As the inorganic compound, sodium chloride, calcium chloride, iron chloride, magnesium sulfate, iron sulfate, manganese sulfate, calcium carbonate, or the like may be used, and in addition, amino acids, vitamins, and/or suitable precursors may be included. These components or precursors may be added to the medium in a batch or continuous mode. However, it is not limited thereto.

During the cultivation of the microorganism, compounds such as ammonium hydroxide, potassium hydroxide, ammonia, phosphoric acid, sulfuric acid, or the like may be added to the medium in an appropriate manner to adjust the pH of the medium.

In the culturing of the present disclosure, the pH in the culture medium may be 5 to 9, 5 to 8.5, 5 to 8, 5 to 7.5, 5 to 7, 5 to 6.5, 5 to 6, 5 to 5.5, 5.5 to 9, 5.5 to 8.5, 5.5 to 8, 5.5 to 7.5, 5.5 to 7, 5.5 to 6.5, 5.5 to 6, 6 to 9, 6 to 8.5, 6 to 8, 6 to 7.5, 6 to 7, 6 to 6.5, 6.5 to 9, 6.5 to 8.5, 6.5 to 8, 6.5 to 7.5, 6.5 to 7, 7 to 9, 7 to 8.5, 7 to 8, 7 to 7.5, 7.5 to 9, 7.5 to 8.5, 7.5 to 8, 8 to 9, 8 to 8.5, or 8.5 to 9, but is not limited thereto. In one embodiment, the pH in the culture medium may be 6 to 8, or 7, but is not limited thereto.

In addition, in order to maintain the aerobic condition of the medium, oxygen or an oxygen-containing gas may be injected into the medium, or in order to maintain anaerobic and microaerobic conditions, gas injection may be omitted or nitrogen, hydrogen, or carbon dioxide gas may be injected, but is not limited thereto. In one embodiment, the oxygen concentration in the medium may be maintained in a range in which dissolved oxygen is not limited during cultivation of the microorganism so that the microorganism may grow normally. In one embodiment, the oxygen concentration in the medium may be 0.1 to 20 ppm, but is not limited thereto. Air may be supplied to the medium at an appropriate amount and rate so that the oxygen concentration in the medium may be maintained in the range. In one embodiment, air may be supplied to the medium at 0.1 to 2 vvm (volume of air per volume of medium per minute) so that the oxygen concentration in the medium may be maintained in the range, but is not limited thereto.

In the culturing of the present disclosure, the culture temperature may be maintained at 20 to 45 °C, specifically 25 to 40 °C, and culturing may be performed for about 10 to 160 hours, but is not limited thereto.

Lysine produced by the culturing of the present disclosure may be secreted into the medium or remain in the cells. The method for producing lysine or the method for increasing lysine production of the present disclosure may further include a step of recovering lysine from the medium resulting from the culturing (medium in which culturing was performed) or microorganism (e.g., strain of the genus *Corynebacterium*). The recovering step may be further included after the culturing step.

The recovery may be collecting the desired lysine using a suitable method known in the technical field according to the culture method of the microorganism of the present disclosure, for example, a batch, continuous, or fed-batch culture method. For example, various methods such as centrifugation, filtration, crystallization, treatment with a protein precipitant (salting out method), extraction, ultrasonic disruption, ultrafiltration, dialysis, molecular sieve chromatography (gel filtration), adsorption chromatography, ion exchange chromatography, affinity chromatography, etc., HPLC, or a combination of these methods may be used, and the desired lysine may be recovered from the medium or microorganism using a suitable method known in the field.

In addition, the method for producing lysine or the method for increasing lysine production of the present disclosure may additionally include a step of purifying lysine. The purification may be performed using a suitable method known in the technical field. In one embodiment, when the method for producing lysine or the method for increasing lysine production of the present disclosure includes both the recovery step and the purification step, the recovery step and the purification step may be performed continuously or discontinuously regardless of the order, or may be performed simultaneously or integrated into one step, but are not limited thereto.

Another aspect is to provide a composition for producing lysine comprising the microorganism of the genus *Corynebacterium*, a medium in which the microorganism has been cultured, or a combination thereof.

The composition may further comprise any suitable excipient commonly used in compositions for producing lysine, and such an excipient may be, for example, a preservative, a wetting agent, a dispersant, a suspending agent, a buffer, a stabilizer, or an isotonic agent, but is not limited thereto.

Another aspect provides the use of the microorganism of the genus *Corynebacterium* for producing lysine, for increasing the production thereof, or for the manufacture of a composition for producing lysine.

The present disclosure is summarized as follows:
1. A lysine-producing microorganism of the genus *Corynebacterium*, into which an E. coli-derived cytoplasmic trehalase (treF) is introduced.
2. The microorganism of the genus *Corynebacterium* according to item 1, wherein the E. coli-derived cytoplasmic trehalase comprises an amino acid sequence having at least 94% identity to the amino acid sequence of SEQ ID NO: 1.
3. The microorganism of the genus *Corynebacterium* according to item 1 or 2, wherein the E. coli-derived cytoplasmic trehalase is encoded by a gene comprising the nucleic acid sequence of SEQ ID NO: 2.
4. The microorganism of the genus *Corynebacterium* according to any one of the preceding items, into which an E. coli-derived periplasmic trehalase (treA) is further introduced.
5. The microorganism of the genus *Corynebacterium* according to item 4, wherein the E. coli-derived periplasmic trehalase comprises an amino acid sequence having at least 85% identity to the amino acid sequence of SEQ ID NO: 3.
6. The microorganism of the genus *Corynebacterium* according to item 4 or 5, wherein the E. coli-derived periplasmic trehalase is encoded by a gene comprising the nucleic acid sequence of SEQ ID NO: 4.
7. The microorganism of the genus *Corynebacterium* according to any one of the preceding items, wherein the microorganism of the genus *Corynebacterium* is *Corynebacterium glutamicum*.
8. The microorganism of the genus *Corynebacterium* according to any one of the preceding items, wherein the microorganism has an increased lysine production ability compared to a parent strain or a wild-type microorganism into which the E. coli-derived cytoplasmic trehalase is not introduced.
9. The microorganism of the genus *Corynebacterium* according to any one of the preceding items, which has increased trehalose degradation ability compared to a parent strain or a wild-type microorganism into which the E. coli-derived cytoplasmic trehalase is not introduced.
10. A composition for producing lysine, comprising the microorganism of the genus *Corynebacterium* according to any one of the preceding items.
11. A method for producing lysine, comprising culturing the microorganism of the genus *Corynebacterium* according to any one of the preceding items in a medium.
12. The method for producing lysine according to item 11, further comprising recovering lysine from the medium resulting from the culturing or from the microorganism of the genus *Corynebacterium*.
13. A method for increasing lysine production, comprising culturing the microorganism of the genus *Corynebacterium* according to any one of the preceding items in a medium.

### [ADVANTAGEOUS EFFECTS]

The microorganism into which a trehalase has been introduced according to the present disclosure has excellent lysine production ability and a byproduct-reducing effect, and thus may be efficiently utilized for the mass production of lysine.

### [MODE FOR INVENTION]

Hereinafter, the present disclosure will be described in more detail by the following examples. However, these are only for illustrating the present disclosure, and the scope of the present disclosure is not limited by these examples.

### Example 1. Preparation of a plasmid for gene insertion

To insert the E. coli-derived treF and treA genes into the chromosome of *Corynebacterium glutamicum*, NCgl2284, which is known as a gene encoding a transposon, was used as an insertion site. In order to replace the NCgl2284 gene with E. coli treF and/or treA, an NCgl2284 deletion and target gene insertion vector was prepared. To prepare the vector, PCR was performed using the chromosome of *Corynebacterium glutamicum* ATCC13032 as a template and the primer pairs of SEQ ID NO: 5 and SEQ ID NO: 6, and SEQ ID NO: 7 and SEQ ID NO: 8, respectively. The primer sequences used to perform each PCR are as shown in Table 1 below.

Solg^{™} Pfu-X DNA Polymerase (Solgent) was used as a polymerase for the PCR reaction. The PCR conditions were as follows: denaturation at 95 °C for 30 seconds, denaturation at 55 °C for 30 seconds, and polymerization at 68 °C for 1 minute; these denaturation, annealing, and polymerization reactions were repeated 28 times. As a result, 500-bp DNA fragments were obtained. The obtained DNA products were purified using a PCR purification kit (QIAGEN), and the purified amplification products were cloned into a pDC24 vector (Korean Patent Publication No. KR 10-2024-0167588 A), which had been treated with the restriction enzyme SmaI at 37 °C for 1 hour and then heat-treated at 65 °C for 20 minutes, using an In-Fusion Cloning Kit (TaKaRa) according to the manufacturer's manual, thereby preparing the vector pDC24_△NCgl2284 for NCgl2284 deletion and target gene insertion.

### Example 2. Preparation of a recombinant plasmid for introduction of E. coli-derived treF and treA

To introduce the E. coli-derived treF and treA genes, a plasmid for introducing the E. coli treF and treA genes was prepared using the gapA promoter (PgapA) of *Corynebacterium glutamicum*.

Specifically, a PgapA promoter fragment was obtained by performing PCR using the chromosome of *Corynebacterium glutamicum* ATCC13032 containing the PgapA nucleic acid sequence as a template and using a primer pair of SEQ ID NO: 9 and SEQ ID NO: 10. A treF (protein sequence: SEQ ID NO: 1; gene sequence: SEQ ID NO: 2) fragment was obtained by performing PCR using the E. coli (Escherichia coli str. K-12 substr. MG1655) chromosome (GenBank: CP032667.1) as a template and using SEQ ID NO: 11 and SEQ ID NO: 12 as a primer pair. In addition, a treA (protein sequence: SEQ ID NO: 3; gene sequence: SEQ ID NO: 4) fragment was obtained by performing PCR using SEQ ID NO: 13 and SEQ ID NO: 14 as a primer pair. PfuUltra^{TM} high-fidelity DNA polymerase (Stratagene) was used as a polymerase for the PCR reaction. The PCR conditions were 95 °C for 30 seconds for denaturation, 55 °C for 30 seconds for denaturation, and 68 °C for 3 minutes for polymerization, and the denaturation, annealing, and polymerization reactions were repeated 28 times. As a result, a 488-bp DNA fragment of the PgapA promoter region, a 1650-bp DNA fragment of the E. coli treF region, and a 1698-bp DNA fragment of the treA region were each obtained.

Each obtained PCR fragment was cloned with the pDC24_△NCgl2284 vector, which had been treated with the restriction enzyme SmaI at 37 °C for 1 hour and then heat-treated at 65 °C for 20 minutes, using an In-Fusion Cloning Kit (TaKaRa) according to the provided manual, thereby preparing two types of vectors: pDC24_△NCgl2284::PgapA_treF(E.co) and pDC24_△NCgl2284::PgapA_treA(E.co).

For simultaneous expression of E. coli-derived treF and treA, a PgapA_treF(E.co) fragment was obtained by performing PCR using the previously prepared pDC24_△NCgl2284::PgapA_treF(E.co) as a template with SEQ ID NO: 9 and SEQ ID NO: 15, and a PgapA_treA(E.co) fragment was obtained by performing PCR using pDC24_△NCgl2284::PgapA_treA(E.co) as a template with SEQ ID NO: 14 and SEQ ID NO: 16. The amplification products were purified using a PCR purification kit (QIAGEN) and used as insertion DNA fragments for vector construction. Each obtained PCR fragment was cloned with the pDC24_△NCgl2284 vector, which had been treated with the restriction enzyme SmaI at 37 °C for 1 hour and then heat-treated at 65 °C for 20 minutes, using an In-Fusion Cloning Kit (TaKaRa) according to the provided manual, thereby preparing pDC24_△NCgl2284::PgapA_treF-PgapA(E.co)_treA(E.co). The primer sequences used to perform each PCR and the E. coli-derived treF and treA sequences are shown in Table 1 below.

**[Table 1]**

| **SEQ ID NO** | **Name** | **Sequence** |
|---|---|---|
| 1 | treF(EC) | MLNQKIQNPNPDELMIEVDLCYELDPYELKLDEMIEAPEPEPEMIEGLPASDALTPAD RYLELFEHVQSAKIFPDSKTFPDCAPKMDPLDILIRYRKVRRHRDFDLRKFVENHFW LPEVYSSEYVSDPQNSLKEHIDQLWPVLTREPQDHIPWSSLLALPQSYIVPGGRFSET YYWDSYFTMLGLAESGREDLLKCMADNFAWMIENYGHIPNGNRTYYLSRSQPPVF ALMVELFEEDGVRGARRYLDHLKMEYAFWMDGAESLIPNQAYRHVVRMPDGSLL NRYWDDRDTPRDESWLEDVETAKHSGRPPNEVYRDLRAGAASGWDYSSRWLRD TGRLASIRTTQFIPIDLNAFLFKLESAIANISALKGEKETEALFRQKASARRDAVNRYL WDDENGIYRDYDWRREQLALFSAAAIVPLYVGMANHEQADRLANAVRSRLLTPG GILASEYETGEQWDKPNGWAPLQWMAIQGFKMYGDDLLGDEIARSWLKTVNQF YLEQHKLIEKYHIADGVPREGGGGEYPLQDGFGWTNGVVRRLIGLYGEP |
| 2 | treF(EC) | ATGCTCAATCAGAAAATTCAAAACCCTAATCCAGACGAACTGATGATCGAAGTCG ATCTCTGCTATGAGCTGGACCCGTATGAATTAAAACTGGATGAGATGATCGAGGC AGAACCGGAACCCGAGATGATTGAAGGGCTGCCTGCCTCTGATGCGCTGACGCC TGCCGATCGCTATCTCGAACTGTTCGAGCATGTTCAGTCGGCGAAAATTTTCCCCG ACAGTAAAACCTTTCCCGACTGCGCACCTAAAATGGACCCGCTGGATATCTTAATC CGCTACCGTAAAGTGCGCCGTCATCGTGATTTTGACTTGCGCAAGTTTGTTGAAA ACCACTTCTGGCTGCCGGAGGTCTACTCCAGCGAGTATGTATCGGACCCGCAAAA TTCCCTGAAAGAGCATATCGACCAGCTGTGGCCGGTGCTAACCCGCGAACCACA GGATCACATTCCGTGGTCTTCTCTGCTGGCGCTGCCGCAGTCATATATTGTCCCGG GCGGCCGTTTTAGCGAAACCTACTATTGGGATTCCTATTTCACCATGCTGGGGCTG GCGGAAAGTGGTCGGGAAGATTTGCTGAAATGCATGGCCGATAACTTCGCCTGG ATGATCGAAAACTACGGTCACATCCCCAACGGCAACCGCACCTATTATTTGAGCC GCTCGCAACCACCGGTTTTTGCGCTGATGGTGGAGTTGTTTGAAGAAGATGGTGT ACGCGGTGCGCGCCGCTATCTCGACCACCTTAAAATGGAATATGCCTTCTGGATG GACGGTGCAGAATCGTTAATCCCTAATCAGGCCTATCGCCATGTTGTGCGGATGCC GGACGGATCGCTGCTCAACCGTTACTGGGACGATCGCGACACGCCGCGTGACGA ATCCTGGCTTGAGGACGTTGAAACCGCGAAACATTCTGGTCGCCCGCCCAACGA GGTGTACCGCGATTTACGCGCGGGGGCGGCCTCCGGTTGGGATTACTCTTCCCGT TGGCTGCGTGATACTGGTCGTCTGGCGAGCATTCGTACCACCCAGTTCATCCCCAT CGATCTGAATGCCTTCCTGTTTAAACTGGAGAGCGCCATCGCCAACATCTCGGCG CTGAAAGGCGAGAAAGAGACAGAAGCACTGTTCCGCCAGAAAGCCAGTGCCCG TCGCGATGCGGTAAACCGTTACCTCTGGGATGATGAAAACGGCATCTACCGCGAT TACGACTGGCGACGCGAACAACTGGCGCTGTTTTCCGCTGCCGCCATTGTGCCAC TCTATGTCGGTATGGCGAACCATGAACAGGCCGATCGTCTGGCAAACGCCGTGCG CAGTCGGTTACTGACACCTGGCGGGATTCTGGCAAGCGAGTACGAAACCGGTGA ACAGTGGGATAAACCCAACGGCTGGGCACCGTTACAATGGATGGCGATTCAGGG ATTTAAAATGTACGGCGATGACCTTCTGGGTGATGAAATCGCGCGAAGCTGGCTG AAGACGGTGAATCAGTTCTATCTGGAACAGCACAAACTGATCGAAAAATACCATA TTGCCGATGGTGTTCCCCGCGAAGGCGGCGGTGGCGAGTATCCGTTGCAGGATG GGTTTGGCTGGACTAACGGTGTGGTACGCCGTTTAATTGGTTTGTACGGCGAACC ATAA |
| 3 | treA(EC) | MKSPAPSRPQKMALIPACIFLCFAALSVQAEETPVTPQPPDILLGPLFNDVQNAKLFI DQKTFADAVPNSDPLMILADYRMQQNQSGFDLRHFVNVNFTLPKEGEKYVPPEG QSLREHIDGLWPVLTRSTENTEKWDSLLPLPEPYVVPGGRFREVYYWDSYFTMLGL AESGHWDKVADMVANFAHEIDTYGHIPNGNRSYYLSRSQPPFFALMVELLAQHEG DAALKQYLPQMQKEYAYWMDGVENLQAGQQEKRVVKLQDGTLLNRYWDDRDT PRPESWVEDIATAKSNPNRPATEIYRDLRSAAASGWDFSSRWMDNPQQLNTLRTT SIVPVDLNSLMFKMEKILARASKAAGDNAMANQYETLANARQKGIEKYLWNDQQ GWYADYDLKSHKVRNQLTAAALFPLYVNAAAKDRANKMATATKTHLLQPGGLNT TSVKSGQQWDAPNGWAPLQWVATEGLQNYGQKEVAMDISWHFLTNVQHTYDF EKKLVEKYDVSTTGTGGGGGEYPLQDGFGWTNGVTLKMLDLICPKEQPCDNVPAT RPTVKSATTQPSTKEAQPTP |
| 4 | treA(EC) | ATGAAATCCCCCGCACCTTCTCGCCCGCAAAAAATGGCGTTAATTCCAGCCTGTAT CTTTTTGTGTTTCGCTGCGCTATCGGTGCAGGCAGAAGAAACACCGGTAACACCA CAGCCGCCTGATATTTTATTAGGGCCGCTGTTTAATGATGTGCAAAACGCCAAACT TTTTCCGGACCAAAAAACCTTTGCCGATGCCGTGCCGAACAGCGATCCGCTGATG ATCCTTGCTGATTATCGGATGCAGCAAAACCAGAGCGGATTTGATCTGCGCCATTT CGTTAACGTCAATTTCACCCTGCCGAAAGAAGGCGAGAAATATGTTCCGCCAGAG GGGCAGTCACTGCGCGAACATATTGACGGACTTTGGCCGGTATTAACGCGTTCTA CCGAAAACACCGAAAAATGGGATTCTCTGTTACCGCTGCCGGAACCTTATGTCGT GCCGGGCGGACGCTTTCGCGAGGTATATTACTGGGACAGTTACTTCACCATGTTA GGACTTGCCGAAAGCGGTCACTGGGATAAAGTCGCGGATATGGTGGCCAATTTT GCTCATGAAATAGACACTTACGGTCATATTCCCAACGGCAACCGCAGTTACTATTT AAGCCGCTCGCAACCGCCCTTCTTTGCCCTGATGGTAGAGTTACTGGCGCAGCAT GAAGGCGATGCCGCGTTGAAGCAATACCTGCCGCAAATGCAAAAAGAATATGCT TACTGGATGGACGGTGTTGAAAACCTGCAAGCCGGACAACAGGAAAAACGCGT TGTCAAACTTCAGGATGGTACCCTTCTCAACCGCTACTGGGACGATCGCGATACG CCACGACCAGAGTCATGGGTGGAAGATATTGCCACCGCCAAAAGCAATCCGAAT CGACCTGCCACTGAAATTTACCGCGACCTGCGCTCTGCCGCTGCGTCTGGCTGGG ATTTCAGCTCGCGCTGGATGGACAACCCGCAGCAGTTAAATACCTTACGCACCAC CAGCATCGTACCGGTCGATCTGAACAGCCTGATGTTTAAAATGGAAAAAATCCTC GCCCGCGCCAGCAAAGCTGCCGGAGATAACGCGATGGCAAACCAGTACGAAAC GCTGGCAAATGCCCGTCAAAAAGGGATCGAAAAATACCTGTGGAACGATCAACA AGGCTGGTATGCCGATTACGACCTGAAAAGTCATAAAGTGCGCAATCAGTTAACC GCGGCCGCCCTGTTCCCGCTGTACGTCAATGCGGCAGCGAAAGATCGCGCCAAC AAAATGGCGACGGCGACGAAAACACATCTGCTGCAACCCGGCGGCCTGAACAC CACGTCGGTGAAAAGTGGGCAACAATGGGATGCGCCAAATGGCTGGGCACCGT TACAGTGGGTCGCGACAGAAGGATTACAAAACTACGGGCAAAAAGAGGTGGCG ATGGACATTAGCTGGCACTTCCTGACCAATGTTCAGCACACCTATGACCGGGAGA AAAAGCTGGTGGAAAAATATGATGTCAGCACCACCGGAACGGGGGGCGGCGGT GGCGAATATCCATTACAGGATGGCTTTGGCTGGACCAATGGCGTGACGCTGAAA ATGCTGGATTTGATCTGCCCGAAAGAGCAACCGTGTGACAATGTTCCGGCGACGC GTCCGACCGTTAAGTCAGCAACGACGCAACCCTCAACCAAAGAGGCACAACCC ACACCTTAA |
| 5 | Primer | TCGAGCTCGGTACCCCTTGTATGGCTCGGGGAAGAACTTA |
| 6 | Primer | AACCTCCCCTCATATGCAGAAATCCACATCAATCCAGGTG |
| 7 | Primer | GTGGATTTCTGCATATGAGGGGAGGTTTGACCAAATAGCG |
| 8 | Primer | CTCTAGAGGATCCCCTCACAGTGCGAAGACGGCGCCAACG |
| 9 | Primer | GTGGATTTCTGCATAATGATTGAAGCCTAAAAACGACCGA |
| 10 | Primer | GTTGTGTCTCCTCTAAAGATTGTAG |
| 11 | Primer | TAGAGGAGACACAACATGCTCAATCAGAAAATTCAAAACC |
| 12 | Primer | AAACCTCCCCTCATATTATGGTTCGCCGTACAAACCAATT |
| 13 | Primer | TAGAGGAGACACAACATGAAATCCCCCGCACCTTCTCGCC |
| 14 | Primer | AAACCTCCCCTCATATTAAGGTGTGGGTTGTGCCTCTTTG |
| 15 | Primer | TTAGGCTTCAATCATTTATGGTTCGCCGTACAAACCAATT |
| 16 | Primer | ATGATTGAAGCCTAAAAACGACCGA |

### Example 3. Construction of a strain into which E. coli treF and treA are introduced and evaluation of lysine production ability

The three types of vectors (pDC24_△NCgl2284::PgapA_treF(E.co), pDC24_△NCgl2284::PgapA_treA(E.co), pDC24_△NCgl2284::PgapA_treF-PgapA(E.co)_treA(E.co)) prepared in Example 2 were transformed into *Corynebacterium glutamicum* CJ3P (US 9556463 B2), which is a lysine-producing strain, by electroporation using an electric pulse method (Van der Rest et al., Appl. Microbiol. Biotecnol. 52:541-545, 1999), and each strain was named CJ3P_treF(E.co), CJ3P_treA(E.co), and CJ3P_treF-treA(E.co).

In order to confirm the L-lysine production ability and trehalose degradation ability of the prepared strains and the parent strain as a control group, flask fermentation titer evaluation was performed.

First, each strain was inoculated into a 250 ml corner-baffle flask containing 25 ml of seed medium, and shaking culture was performed at 37 °C for 20 hours at 200 rpm. 1 ml of seed culture was inoculated into a 250 ml corner-baffle flask containing 24 ml of production medium, and shaking culture was performed at 37 °C for 40 hours at 200 rpm.

The CJ3P strain, which is a parent strain, and CJ3P_treF(E.co), CJ3P_treA(E.co), and CJ3P_treF-treA(E.co) strains, into which E. coli-derived treF and treA were introduced, were cultured in flasks using a production medium, and lysine concentration and trehalose concentration were measured. The experiment was repeated three times, and average values of the analysis results are shown in Table 2 below.

### <Seed medium>

20 g of raw sugar, 10 g of peptone, 5 g of yeast extract, 1.5 g of urea, 4 g of KH₂PO₄, 8 g of K₂HPO₄, 0.5 g of MgSO_{4 ·}7H₂O, 0.1 mg of biotin, 1 mg of thiamine HCl, 2 mg of calcium pantothenate, and 2 mg of nicotinamide (based on 1 liter of distilled water)

### <Production medium>

100 g of raw sugar, 40 g of (NH₄)₂SO₄, 2.5 g of soy protein, 5 g of corn steep solids, 3 g of urea, 1 g of KH₂PO₄, 0.5 g of MgSO_{4·}7H₂O, 100 µg of biotin, 1000 µg of thiamine hydrochloride, 2000 µg of calcium pantothenate, 3000 µg of nicotinamide, and 30 g of CaCO₃ (based on 1 liter of distilled water).

**[Table 2]**

| Strain | 40hr | | | | |
|---|---|---|---|---|---|
| | Cell mass (OD₆₀₀ₙₘ) | Lysine concentration (g/L) | Lysine concentration Increase rate (%) | Trehalose (mg/L) | Trehalose concentration Reduction rate (%) |
| CJ3P | 43.5 | 6.21 | 100 | 138.5 | 0.0 |
| CJ3P_treF(E.co) | 45.7 | 7.11 | 114.5 | 61.5 | 55.6 |
| CJ3P_treA(E.co) | 44.2 | 6.96 | 112.1 | 71.7 | 48.2 |
| CJ3P_treF-treA(E.co) | 45.1 | 7.47 | 120.3 | 3.2 | 97.7 |

As shown in Table 2 above, it was confirmed that the lysine concentration of the CJ3P_treF(E.co) strain, into which E. coli-derived treF was introduced, and the CJ3P_treA(E.co) strain, into which E. coli-derived treA was introduced, increased by approximately 14.5% and 12.1%, respectively, compared to the parent strain, and it was confirmed that the trehalose accumulated in the medium decreased by 55.6% and 48.2%, respectively, due to the introduction of the genes. In addition, for the CJ3P_treF-treA(E.co) strain into which both genes were introduced, the lysine concentration increased by approximately 20.3% compared to the parent strain, and the trehalose in the medium was confirmed to decrease by up to 97.7%. Based on the above results, it was confirmed that the introduction of E. coli-derived treF not only increases the lysine concentration but also decreases the trehalose accumulated in the medium. Furthermore, it was confirmed that the simultaneous introduction of E. coli-derived treF and treA reduces trehalose more effectively, and it is considered that the reduction of trehalose in the medium contributes to the improvement of fermentation purity.

From the above description, a person skilled in the art to which the present invention belongs will be able to understand that the present disclosure may be implemented in other specific forms without changing its technical spirit or essential features. In this regard, it should be understood that the embodiments described above are illustrative in all respects and not restrictive. The scope of the present disclosure should be construed such that all changes or modified forms derived from the meaning and scope of the following claims and their equivalent concepts, rather than the above detailed description, are included in the scope of the present disclosure.

## Claims

1. A lysine-producing microorganism of the genus *Corynebacterium*, into which an E. coli-derived cytoplasmic trehalase (treF) is introduced.

2. The microorganism of the genus *Corynebacterium* according to claim 1, wherein the E. coli-derived cytoplasmic trehalase comprises an amino acid sequence having at least 94% identity to the amino acid sequence of SEQ ID NO: 1.

3. The microorganism of the genus *Corynebacterium* according to claim 1, wherein the E. coli-derived cytoplasmic trehalase is encoded by a gene comprising the nucleic acid sequence of SEQ ID NO: 2.

4. The microorganism of the genus *Corynebacterium* according to claim 1, into which an E. coli-derived periplasmic trehalase (treA) is further introduced.

5. The microorganism of the genus *Corynebacterium* according to claim 4, wherein the E. coli-derived periplasmic trehalase comprises an amino acid sequence having at least 85% identity to the amino acid sequence of SEQ ID NO: 3.

6. The microorganism of the genus *Corynebacterium* according to claim 4, wherein the E. coli-derived periplasmic trehalase is encoded by a gene comprising the nucleic acid sequence of SEQ ID NO: 4.

7. The microorganism of the genus *Corynebacterium* according to claim 1, wherein the microorganism of the genus *Corynebacterium* is *Corynebacterium glutamicum*.

8. The microorganism of the genus *Corynebacterium* according to claim 1, wherein the microorganism has an increased lysine production ability compared to a parent strain or a wild-type microorganism into which the E. coli-derived cytoplasmic trehalase is not introduced.

9. The microorganism of the genus *Corynebacterium* according to claim 1, wherein the microorganism has an increased trehalose degradation ability compared to a parent strain or a wild-type microorganism into which the E. coli-derived cytoplasmic trehalase is not introduced.

10. A composition for producing lysine, comprising the microorganism of the genus *Corynebacterium* according to any one of claims 1 to 9.

11. A method for producing lysine, comprising culturing the microorganism of the genus *Corynebacterium* according to any one of claims 1 to 9 in a medium.

12. The method for producing lysine according to claim 11, further comprising recovering lysine from the medium resulting from the culturing or from the microorganism of the genus *Corynebacterium*.

13. A method for increasing lysine production, comprising culturing the microorganism of the genus *Corynebacterium* according to any one of claims 1 to 9 in a medium.
